# EUROPÄISCHE PATENTSCHRIFT

(11) **EP 1 354 562 B1**
(45) Veröffentlichungstag und Bekanntmachung des Hinweises auf die Patenterteilung: **07.09.2005**
(21) Anmeldenummer: 02008727.6
(22) Anmeldetag: 18.04.2002
(51) Int. Cl.: A61B 17/58

(54) **Knochenfixierungssystem**
Improved bone fixation
Système d' ostéosynthèse

(43) Veröffentlichungstag der Anmeldung: 22.10.2003
(73) Patentinhaber: Zimmer GmbH, 8404 Winterthur (CH)
(72) Erfinder: Rööser, Bo, 43243 Varberg (SE); Schwammberger, Andreas Eduard, 4434 Hölstein (CH)
(74) Vertreter: Manitz, Finsterwald & Partner GbR

(56) Entgegenhaltungen:
- EP-A- 0 321 170
- EP-A- 0 640 318
- WO-A-00/38586
- US-A- 4 827 917

## Beschreibung

Die Erfindung betrifft ein Knochenfixierungssystem, das bei der operativen Versorgung von Frakturen einsetzbar ist.

Je nach Art und Schwere einer Fraktur kann es erforderlich sein, diese operativ zu behandeln. Gängige Verfahren hierzu sind unter anderem geschlossene Reposition, d.h. Ausrichtung der Knochenfragmente in ihre korrekte Lage, mit nachfolgender Einbohrung eines oder mehrerer Stifte aus rostfreiem Stahl über die Fraktur unter Röntgenkontrolle. Gegebenenfalls kann noch ein Gipsverband angelegt werden. Es können auch Gewindestifte verwendet werden, die mittels eines Rahmens außerhalb der Haut verbunden werden, wobei der Rahmen eine Ausrichtung der Fraktur erlaubt.

Manche Frakturen werden unter Freilegung der Fraktur operiert, wobei die Fixierung über eine Metallplatte erfolgt, die mittels Schrauben befestigt wird.

Aus der EP-A-0 321 170 ist eine Vorrichtung bekannt, die zur operativen Behandlung von Oberschenkelhalsbrüchen dient. Die Vorrichtung umfasst einen Schraubenabschnitt und einen mit diesem verbundenen Nagelabschnitt, der Querbohrungen aufweist. Der Nagelabschnitt wird in den Oberschenkelknochen eingeschlagen und der Schraubenabschnitt wird in den Kopf des Oberschenkelknochens eingeschraubt. Anschließend wird der Nagel über Fixierstifte, die durch seine Querbohrungen eingeführt werden, fixiert. Zur Hilfe bei der Fixierung weist die Vorrichtung eine Bohrschablone auf, über die der Operateur die Fixierstifte in die von außen nicht sichtbaren Querbohrungen einführen kann. Diese Vorrichtung ist speziell zur Behandlung von Oberschenkelhalsbrüchen entworfen und für andere Frakturarten wenig geeignet.

Die WO 00 38586 A, die als der nächstliegende Stand der Technik anzusehen ist, betrifft eine Knochenschraube, die Querbohrungen zur Aufnahme von Fixierschrauben aufweist, wobei die Achsen der Knochenschraube und der Fixierschrauben in derselben Ebene liegen.

Es ist die Aufgabe der Erfindung, dem Chirurgen ein Instrument an die Hand zu geben, mit dem eine große Vielfalt von unterschiedlichen Frakturen behandelt werden kann, wobei einerseits eine einfache und sichere Handhabung durch den Chirurgen gewährleistet wird, und andererseits die Fraktur des Patienten zuverlässig in ihre richtige Lage gebracht und gehalten werden kann, so dass ihre Ausheilung unter optimalen Bedingungen erfolgen kann.

Die Aufgabe wird durch das erfindungsgemäße Knochenfixierungssystem mit den Merkmalen des Anspruches 1 gelöst.

Über die Knochenschraube erlaubt das erfindungsgemäße Knochenfixierungssystem, dass zueinander gehörende Knochenfragemente unter Aufbau einer Druckspannung fest in ihrer richtigen Relativlage aneinander gefügt werden können, d.h. eine Schrägstellung und parallele Seitenverschiebung der Fraktur in zwei Ebenen kann vermieden werden. Für das Beibehalten der richtigen Relativlage ist es zweckmäßig, die Knochenschraube drehfest zu machen oder "zu verriegeln". Diese Verriegelungsfunktion übernimmt eine in die Fixierbohrung eingebrachte Schraube oder ein gleichartig anzuwendender Nagel. Ein solches Fixierelement besitzt ein großes winkelstabilisierendes Vermögen, da das Zentrum der Winkelkräfte, die zwischen Fixierelement und Knochenschraube wirken, innerhalb der Knochenschraube wirken und nicht im Knochen selbst.

Das Einführen von beispielsweise einem Nagel als stiftförmiges Fixierelement in die Fixierbohrung der Knochenschraube erfolgt erfindungsgemäß unter Verwendung der Zielhilfe. Da die Zielbohrung im Zielabschnitt exakt mit der Fixierbohrung fluchtet, kann der Nagel über die Zielbohrung von außen her derart eingeführt werden, dass er die Fixierbohrung genau trifft. Der Nagel wird dabei von außen in die Zielbohrung eingeführt, tritt dann aus der Zielbohrung aus, durchdringt anschließend die Haut, die gegebenenfalls durch Ansetzen eines geeigneten Schnittes vorbereitet wird, und durchdringt weitere Gewebeschichten bis zum beschädigten Knochen, dringt in den Knochen ein, bis er in die Fixierbohrung einfädelt, tritt aus dieser aus und gelangt dann wieder in den Knochen. Der gesamte Vorgang wird zweckmäßigerweise über Röntgendurchleuchtung überwacht, um insbesondere die Tiefe des Einführens des Nagels zu kontrollieren.

Die Zielhilfe ist abnehmbar. Sie wird nach Eindrehen der Knochenschraube an deren Kopf befestigt und nach durchgeführter Operation entfernt. Das Fixierelement kann dann kurz über dem Knochen gekürzt werden. Da kein äußerer Rahmen vorgesehen ist und auch kein Gipsverband notwendig ist, können die betreffenen Gliedmaße durch frühzeitiges Training beweglich gehalten werden und ein starker Abbau der Muskulatur kann vermieden werden.

Da die Fixierelemente nahe des Knochens abgeschnitten und die Wunden gleich nach der Operation steril verbunden werden, erfolgt eine vollständig interne Fixierung, die insbesondere das Infektionsrisiko vermindert, das bei Vorrichtungen gegenwärtig ist, die eine Verspannung von Stiften außerhalb des Körpers benutzen und bei denen die Stifte in der gesamten Heilungsphase aus der Haut hervorragen, so dass an der Übergangsstelle zwischen Stift und Haut Keime in die Wunde eindringen können.

Die Verwendung einer Knochenschraube statt eines Nagels ist insbesondere deshalb von Vorteil, da durch das Vorbohren und Einschrauben der Knochenschraube weit weniger Spannungen in dem Knochen aufgebaut werden als beim Einbringen von Nägeln. Dies ist z.B. bei osteoporotischen Knochen günstiger, die dem erhöhten Risiko ausgesetzt sind, dass sie beim Hineintreiben eines Nagels brechen.

Bei einem bevorzugten Ausführungsbeispiel ist die Fixierbohrung als Schrägbohrung ausgebildet, die unter einem spitzen Winkel zur Schraubenachse verläuft, und es ist mindestens eine weitere Fixierbohrung vorgesehen ist, die als Querbohrung ausgebildet ist und rechtwinklig zur Schraubenachse verläuft, wobei der Schrägbohrung und der Querbohrung entsprechend ausgerichtete Zielbohrungen zugeordnet sind. Besonders vorteilhaft ist es, wenn mindestens zwei Fixierbohrungen als Schrägbohrungen ausgebildet sind, die unter einem spitzen Winkel zur Schraubenachse verlaufen und gegeneinander um die Schraubenachse verdreht sind. Diese räumliche Anordnung der Schraube mit den eingebrachten Fixierelementen ergibt eine besonders stabile Verspannung oder Lagefixierung der Schraube im Raum.

Bei einem besonders bevorzugten Ausführungsbeispiel sind die beiden Schrägbohrungen zwischen dem Schraubenkopf und wenigstens einer, bevorzugt mehreren und insbesondere parallel verlaufenden Querbohrungen vorgesehen, wobei diesen Bohrungen entsprechend ausgerichtete Zielbohrungen zugeordnet sind. Besonders vorteilhaft ist es, wenn die beiden Schrägbohrungen nahe dem Schraubenkopf vorgesehen sind und die Querbohrungen im Endbereich des Schaftabschnitts vorgesehen sind. Diese geometrische Konstruktion mit Fixierpunkten in drei Dimensionen trägt zu verbesserter Stabilität der Fixierung der Fraktur bei. Außerdem kann der freie Schenkel der Zielhilfe schlank gehalten werden, so dass er beim operativen Eingriff nicht stört und sein Gewicht möglichst gering bleibt.

Bei einem weiteren Ausführungsbeispiel umfasst die Kopplungseinrichtung des Schraubenkopfes eine in axialer Richtung der Knochenschraube verlaufende Gewindebohrung und eine Positioniereinrichtung in Form einer an der Stirnseite des Schraubenkopfes vorgesehenen Positionierausnehmung, und der Kopplungsabschnitt der Zielhilfe umfasst eine Durchgangsbohrung und eine Positioniereinrichtung in Form einer zur Positionierausnehmung komplementären Positioniererhebung. Besonders bevorzugt ist es, wenn die Positionierausnehmung eine sich radial verjüngende Nut oder zumindest eine durch die Schraubenachse verlaufende kerben- oder schlitzartige Vertiefung umfasst, wobei vorzugsweise zusätzlich zu der kerben- oder schlitzartigen Vertiefung eine randseitig am Schrauben-kopf angeordnete Aussparung ausgebildet ist. Die Zielhilfe ist auf diese Weise einfach über ein standardisiertes Befestigungselement am Schraubenkopf zu befestigen, wobei die Positioniereinrichtungen die Fixierung der Zielhilfe relativ zur Knochenschraube eindeutig festlegen. Dabei ist insgesamt nur eine geringe Anzahl von Einzelteilen für das Knochenfixie-rungssystem erforderlich, nämlich die Knochenschraube, die Zielhilfe, das Befestigungselement und die vorgesehene Anzahl von Fixierelementen.

Die Zielhilfe des erfindungsgemäßen Knochenfixierungssystems ist bei einem weiteren Ausführungsbeispiel als Zielbügel mit einstückig ausgebildetem Kopplungsabschnitt und Zielabschnitt gestaltet. Vorzugsweise liegen der Zielabschnitt und die Knochenschraube im gekoppelten Zustand in einer Ebene und bilden jeweils auseinanderlaufende Schenkel eines U oder V, wobei die Schenkel bevorzugt zumindest näherungsweise gleich lang sind. Diese Ausgestaltung bietet einen kompakten räumlichen Aufbau, der unter einer Vielzahl von anatomischen Bedingungen einsetzbar ist, wie etwa bei Frakturen eines Unterarmknochens in der Nähe des Handgelenks oder Frakturen des äußeren Fußknöchels.

Eine im eingeschraubten Zustand dem Knochen zugewandte Unterseite des Schraubenkopfes ist bei einer weiteren Ausführungsform als an die Form des jeweiligen Knochens angepasste Anlagefläche ausgebildet.

Bei einer anderen Ausführungsform ist in dem Zielabschnitt zumindest eine Aufnahmebohrung vorgesehen, in die eine Zielhülse mit einer darin ausgebildeten Zielbohrung einbringbar, vorzugsweise steckbar, ist, die mit der Fixierbohrung ausgerichtet ist und in Richtung der Fixierbohrung verschiebbar ist. Die Zielhülse verlängert die Zielbohrung in Richtung der Fixierbohrung, wodurch eine noch genauere Führung des Fixierelements zur Fixierbohrung gewährleistet ist.

Ein weiteres Ausführungsbeispiel des erfindungsgemäßen Knochenfixierungssystems zeichnet sich dadurch aus, dass die Zielhilfe zumindest im Bereich des Kopplungsabschnitts aus für Röntgenstrahlen durchlässigem Material, beispielsweise kohlefaserverstärktem Kunststoff, besteht. Eine Durchlässigkeit für Röntgenstrahlung ist zur Röntgenkontrolle während des operativen Eingriffs angebracht, um jederzeit eine gute Sicht auf die zu fixierenden Knochen und die Schraube und die Fixierelemente, die für die Fixierung der Knochen verwendet werden, zu haben.

Eine weitere vorteilhafte Ausführungsform des Knochenfixierungssystems ist durch eine universelle Zielhilfe gekennzeichnet, die mit Knochenschrauben mit unterschiedlichen Längen und/oder unterschiedlichen Anzahlen und Lagen von Fixierbohrungen verwendbar ist. Eine solche Zielhilfe, die für mehrere bzw. unterschiedliche Knochenschrauben geeignet ist, trägt zur Kostensenkung bei und hält das Instrumentarium für die Operation übersichtlicher.

Die Erfindung betrifft außerdem eine Knochenschraube sowie eine Zielhilfe für ein erfindungsgemäßes Knochenfixierungssystem.

Weitere Ausführungsbeispiele der Erfindung sind in den Unteransprüchen, der Beschreibung sowie der Zeichnung angegeben.

Die Erfindung wird im Folgenden beispielhaft anhand der Zeichnung beschrieben. In dieser zeigen:
- Fig. 1: eine Perspektivansicht einer Ausführungsform des erfindungsgemäßen Knochenfixierungssystems,
- Fig. 2: eine Schnittansicht durch das Knochenfixierungssystems von Fig. 1,
- Fig. 3: eine Seitenansicht des Knochenfixierungssystems von Fig. 1,
- Fig. 4: eine Schnittansicht durch eine erfindungsgemäße Knochenschraube,
- Fig. 5: eine Perspektivansicht auf den Kopf einer Knochenschraube,
- Fig. 6: eine Teilschnittansicht durch eine erfindungsgemäße Knochenschraube,
- Fig. 7: ein weiteres Ausführungsbeispiel einer erfindungsgemäßen Knochenschraube die einen Schraubenkopf mit einer konkav ausgebildeten Unterseite aufweist,
- Fig. 8: ein weiteres Ausführungsbeispiel einer erfindungsgemäßen Knochenschraube ohne radial überstehenden Schraubenkopf,
- Fig. 9: ein Anwendungsbeispiel des erfindungsgemäßen Knochenfixierungssystems bei einer Fraktur eines Unterarmknochens in der Nähe des Handgelenks,
- Fig. 10: ein Anwendungsbeispiel ähnlich wie bei Fig. 9 mit abgenommener Zielhilfe und gekürzten Fixierelementen,
- Fig. 11: ein Anwendungsbeispiel des erfindungsgemäßen Knochenfixierungssystems bei einer Fraktur der Ellenbogenspitze,
- Fig. 12: ein Anwendungsbeispiel ähnlich wie bei Fig. 11 mit abgenommener Zielhilfe und gekürzten Fixierelementen,
- Fig. 13: ein Anwendungsbeispiel des erfindungsgemäßen Knochenfixierungssystems bei einer Fraktur des äußeren Fußknöchels,
- Fig. 14: ein Anwendungsbeispiel ähnlich wie bei Fig. 13 mit abgenommener Zielhilfe und gekürzten Fixierelementen,
- Fig. 15: ein Anwendungsbeispiel des erfindungsgemäßen Knochenfixierungssystems bei einer Fraktur im Schultergelenkkopf,
- Fig. 16: ein Anwendungsbeispiel ähnlich wie bei Fig. 15 mit abgenommener Zielhilfe und gekürzten Fixierelementen,
- Fig. 17: ein weiteres Anwendungsbeispiel des erfindungsgemäßen Knochenfixierungssystems bei einer Fraktur im Schultergelenkkopf,
- Fig. 18: ein Anwendungsbeispiel ähnlich wie bei Fig. 17 mit abgenommener Zielhilfe und gekürzten Fixierelementen,
- Fig. 19: ein weiteres Anwendungsbeispiel des erfindungsgemäßen Knochenfixierungssystems bei einer Fraktur eines Unterarmknochens in der Nähe des Handgelenks.

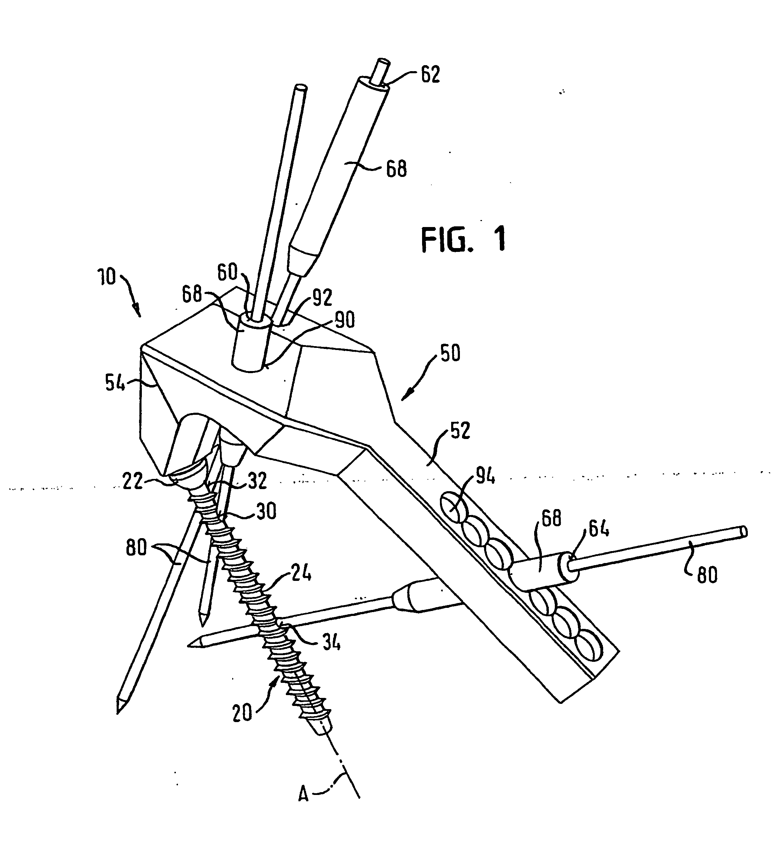

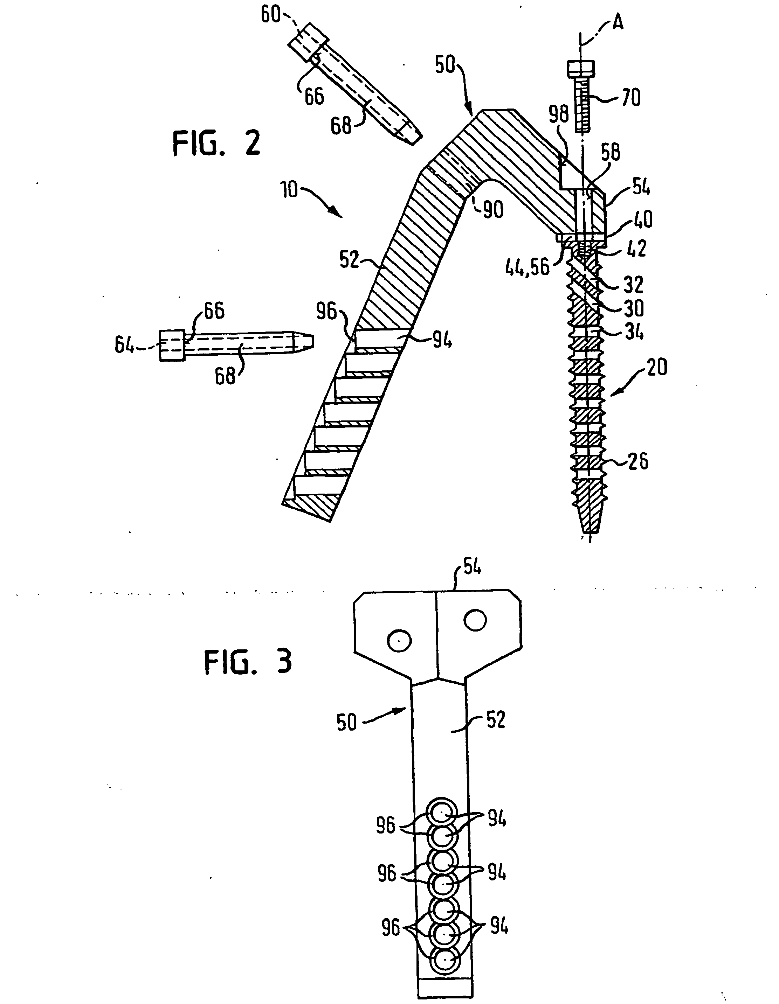

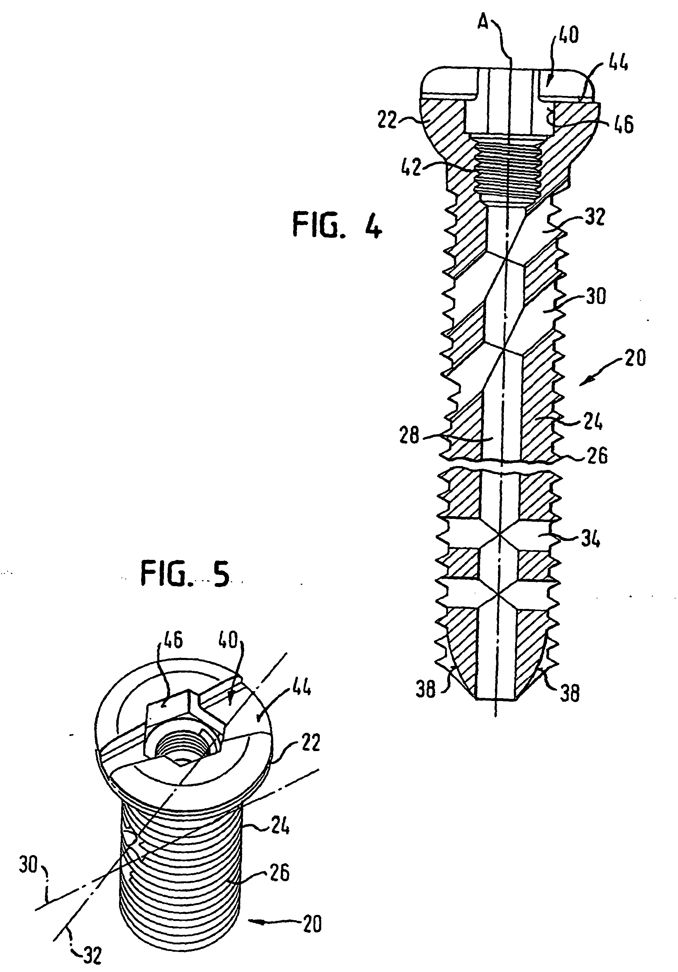

Die Perspektivansicht von Fig. 1 zeigt beispielhaft ein erfindungsgemäßes Knochenfixierungssystem 10, das insbesondere für den Einsatz bei Frakturen von Unterarmknochen in der Nähe des Handgelenks ausgelegt ist. Eine Zielhilfe in der Form eines Zielbügels 50 ist an einem Kopplungsabschnitt 54 über eine Schraube 70 (Fig. 2) an einer Kopplungseinrichtung 40 (Fig. 2 und z.B. Fig. 5) eines Schraubenkopfes 22 einer Knochenschraube 20 befestigt. Der Zielbügel 50 bildet dabei zusammen mit der Knochenschraube 20 die Form eines V mit in einer Ebene liegenden Schenkeln von etwa gleicher Länge, wobei der Öffnungswinkel zwischen den Schenkeln etwa 25° beträgt.

In Aufnahmebohrungen 90, 92, 94 in einem Zielabschnitt 52 des Zielbügels 50 sind Zielhülsen 68 eingesetzt bzw. in einer Position vor Aufnahmebohrungen 92 dargestellt. Durch die Zielhülsen 68 sind Fixierelemente 80 in der Form von Nägeln hindurchgeführt, die sich auch durch Fixierbohrungen 30, 32, 34 hindurch erstrecken, die im Schaftabschnitt 24 und einem an diesem vorgesehenen Außengewindeabschnitt 26 der Knochenschraube 20 ausgebildet sind.

Die beiden in der Nähe des Schraubenkopfes 22 vorgesehenen Fixierbohrungen sind Schrägbohrungen 30, 32, die gegeneinander 22° um die Schraubenachse A verdreht sind und unter einem Winkel von 45° zur Schraubenachse A in einem Abstand von etwa 5 mm zueinander durch die Knochenschraube 20 hindurch verlaufen, so dass die Mittellinien der Schrägbohrungen 30, 32 windschiefe, kreuzungsfreie Geraden bilden. Im unteren Bereich des Schaftabschnitts 24 sind als Querbohrungen 34 ausgebildete Fixierbohrungen vorgesehen, d.h. die Querbohrungen 34 stehen senkrecht auf der Achse A der Knochenschraube 20. Im Zielabschnitt 52 des Zielbügels 50 sind Aufnahmebohrungen 90, 92, 94 für Zielhülsen 68 in einer entsprechenden Anzahl vorgesehen.

Die Knochenschraube 20 spannt zusammen mit jedem in eine entsprechende Fixierbohrung 30, 32, 34 eingeführten Nagel 80 eine Ebene auf, so dass im gezeigten Fall drei Ebenen gebildet sind, die winklig aufeinander stehen und eine stabile geometrische Konstruktion bilden.

Fig. 2 zeigt das Knochenfixierungssystem 10 von Fig. 1 in einer Schnittansicht. Der Kopplungsabschnitt 54 des Zielbügels 50 ist mit der Kopplungseinrichtung 40 am Schraubenkopf 22 der Knochenschraube 20 über eine Schraube 70 verbindbar. Die Kopplungseinrichtung 40 besteht aus einem Innengewinde 42, in das die Schraube 70 eindrehbar ist, und einer Positionierausnehmung 44. Der Kopplungsabschnitt 54 des Zielbügels 50 umfasst ein Durchgangsloch 58 für die Schraube 70 mit einer Flachsenkung 98 zur Aufnahme des Kopfes der Schraube 70 und eine zur Positionierausnehmung 44 komplementäre Positioniererhebung 56. Beim Aufsetzen des Zielbügels 50 auf die Kopplungseinrichtung 40 gelangt die Positioniererhebung 56 des Kopplungsabschnitts 54 in Eingriff mit der Positionierausnehmung 44 der Kopplungseinrichtung 40, so dass nur eine einzige vorgegebene Relativlage zwischen Knochenschraube 20 und Zielbügel 50 hergestellt werden kann und somit eine korrekte Ausrichtung der Zielbohrungen 60, 62, 64 mit ihren zugehörigen Fixierbohrungen 30, 32, 34 gewährleistet wird.

Die Aufnahmebohrungen 90, 92, 94 für die Zielhülse 68 weisen eingangsseitig eine Flachsenkung 96 auf, um eine gute Anlage eines an der Zielhülse 68 vorgesehenen Absatzes 66 am Zielbügel 50 sicherzustellen. Die Zielhülsen 68 sind eintrittsseitig angefast, um sie leicht in die Aufnahmebohrungen 90, 92, 94 einführen zu können.

In Fig. 3 ist zu erkennen, dass der Zielabschnitt 52 im Bereich des Kopplungsabschnitts 54 breiter als an seinem freien Schenkel gestaltet ist, da die beiden zu den Schrägbohrungen 30, 32 gehörenden Aufnahmebohrungen 90, 92, 94 für die Zielhülse 68 in Bezug auf die Mittellinie des Zielbügels 50 seitlich versetzt vorgesehen sind.

Die Schnittansicht von Fig. 4 und die Perspektivansicht von Fig. 5 verdeutlichen im Besonderen die Kopplungseinrichtung 40 einer weiteren Ausführungsform einer Knochenschraube 20. Die Kopplungseinrichtung 40 umfasst die Positionierausnehmung 44, die eine stirnseitig am Schraubenkopf 22 vorgesehene, sich in radialer Richtung verjüngende Nut ist. Zentral in der Stirnseite des Schraubenkopfes 22 ist ein Innensechskant 46 ausgebildet, der als Aufnahme für einen Sechskantschlüssel (nicht gezeigt) als Eindrehwerkzeug dient. Am Ende des Außengewindeabschnitts 26 der Knochenschraube 20 sind gemäß Fig. 4 zwei oder mehr Schneidkanten 38 vorgesehen, da es sich bei der Knochenschraube 20 um eine Schraube mit selbstschneidendem Gewinde handelt, das ggf. lediglich ein Vorbohren, aber keinen separaten Arbeitsgang zum Gewindeschneiden erfordert. Unter bestimmten Umständen, wie etwa bei einem verhältnismäßig kleinen Durchmesser der Knochenschraube 20 in Bezug auf den Knochen, kann auch auf das Vorbohren verzichtet werden. Ferner ist in Achsrichtung durch die gesamte Knochenschraube 20 hindurch eine Durchgangsbohrung 28 vorgesehen, die der Führung dient, wenn vorher ein Kirschner-Draht zur Festlegung der Achslage eingebracht wurde. Ferner sind in Fig. 5 die windschiefen Achsen der Bohrungen 30, 32 veranschaulicht.

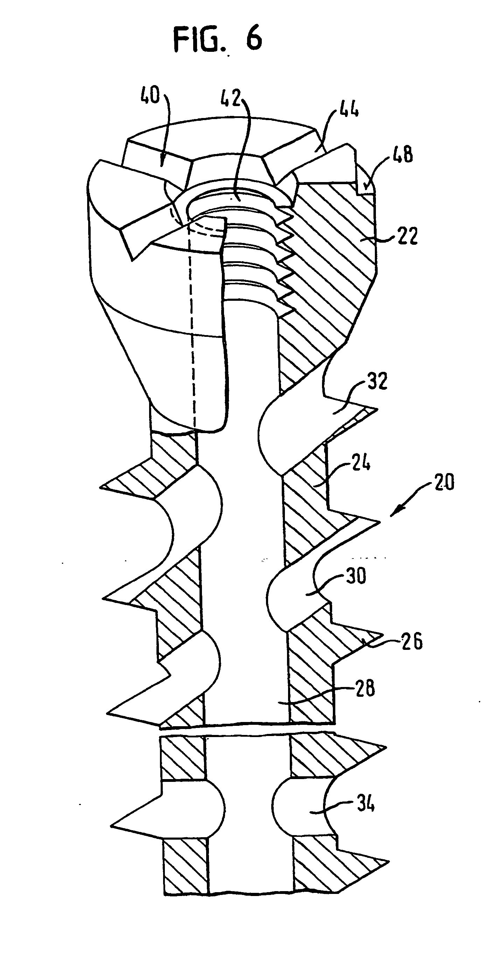

Fig. 6 veranschaulicht ein weiteres Ausführungsbeispiel der erfindungsgemäßen Knochenschraube 20 mit einer andersartig gestalteten Kopplungseinrichtung 40. An der Stirnseite des Schraubenkopfes 22 sind zwei sich in radialer Richtung mittig über den Schraubenkopf 22 hinweg erstreckende Kerben rechtwinklig zueinander vorgesehen, in welchen der Zielbügel mit vorstehenden Dachführungen positioniert wird und an die auch ein Schraubendreher angesetzt werden kann, um bei nicht aufgesetzter Zielhilfe 50 die Knochenschraube 20 in den Knochen einzudrehen. Die Befestigung des Zielbügels 50 in einer einzigen bezüglich Drehung vorgegebenen Relativlage wird dadurch gewährleistet, dass randseitig am Schraubenkopf 22 eine Positionierausnehmung 44 in der Form einer Aussparung 48 ausgebildet ist, mit der eine komplementäre Positioniererhebung 56 des Zielbügels 50 (nicht gezeigt) eine Grobausrichtung im richtigen Quadranten gewährleistet.

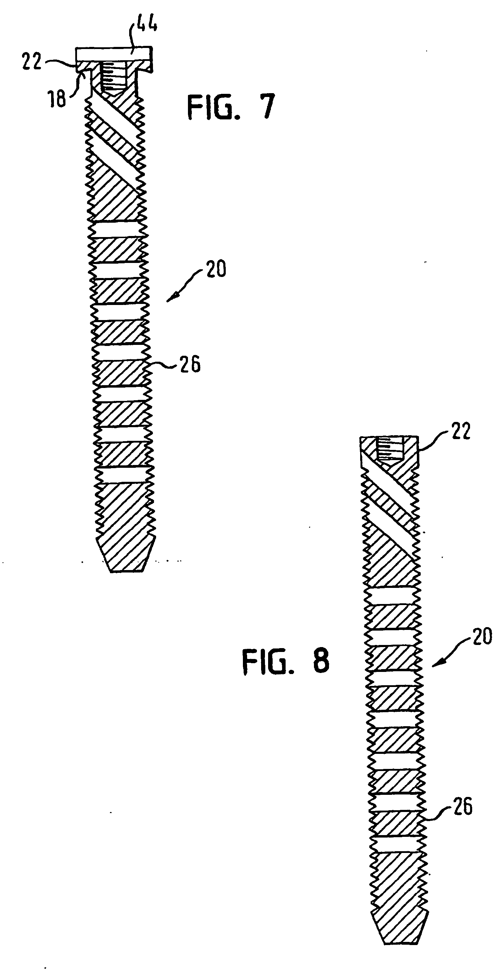

Die schematische Ansicht von Fig. 7 veranschaulicht einen Schraubenkopf eines anderen Ausführungsbeispiels, dessen Unterseite 18 zur Anlage an ein entsprechend geformtes Knochenstück konkav ausgebildet ist.

In Fig. 8 ist ein weiterer Schraubenkopf eines anderen Ausführungsbeispiels einer Knochenschraube dargestellt, der keinen radialen Überstand aufweist und somit z.B. für beengtere Platzverhältnisse geeignet ist.

Weitere Ausführungsformen können noch andere Modifikationen der Knochenschraube 20 aufweisen. Bei sehr kleinen Frakturfragmenten kann es zweckmäßig sein, ausschließlich Querbohrungen 34, die unter 90° zur Schraubenachse stehen, anstelle von Schrägbohrungen zu verwenden.

Die Führungshülsen 68 der Zielhilfe 50 können weggelassen werden, wenn beispielsweise eine Schraube zur Fixierung der Knochenschraube 20 verwendet wird, deren Kopf- und Gewindeteil mit der Bohrung im Zielbügel und in der eigentlichen Knochenschraube übereinstimmen.

Im Folgenden sind Abmessungen eines erfindungsgemäßen Knochenfixierungssystems 10, das beispielhaft zur Behandlung eines Bruches eines Unterarmknochens in der Nähe des Handgelenks eingesetzt wird, angegeben. Die Länge des Schaftabschnitts 24 beträgt zwischen 4 cm und 10 cm und kann in Abhängigkeit von den jeweiligen anatomischen Verhältnissen entsprechend gewählt werden. In dem dargestellten Fall beträgt sie etwa 4 cm. Der Außendurchmesser des Außengewindeabschnitts 26 der Knochenschraube 20 beträgt etwa 6 mm. Das Gewinde des Außengewindeabschnitts 26 der Knochenschraube 20 kann ein eingängiges Gewinde sein, ist aber bevorzugt ein zweigängiges Gewinde mit einer Steigung von etwa 2 mm pro Gang. Die Fixierbohrungen 30, 32, 34 haben einen Durchmesser von 1,6 mm.

Die Knochenschraube 20 und die Fixierelemente bestehen vorzugsweise aus Titan, um eine galvanische Reaktion zwischen verschiedenen Metallen zu vermeiden. Für die Knochenschraube 20 kann aber auch ein vom Körper wieder aufnehmbares Material, beispielsweise Polyglykolid oder Poly-L-Laktid, in Betracht gezogen werden. Die Zielhilfe 50 besteht aus kohlefaserverstärktem Kunststoff und ist daher für Röntgenstrahlen durchlässig.

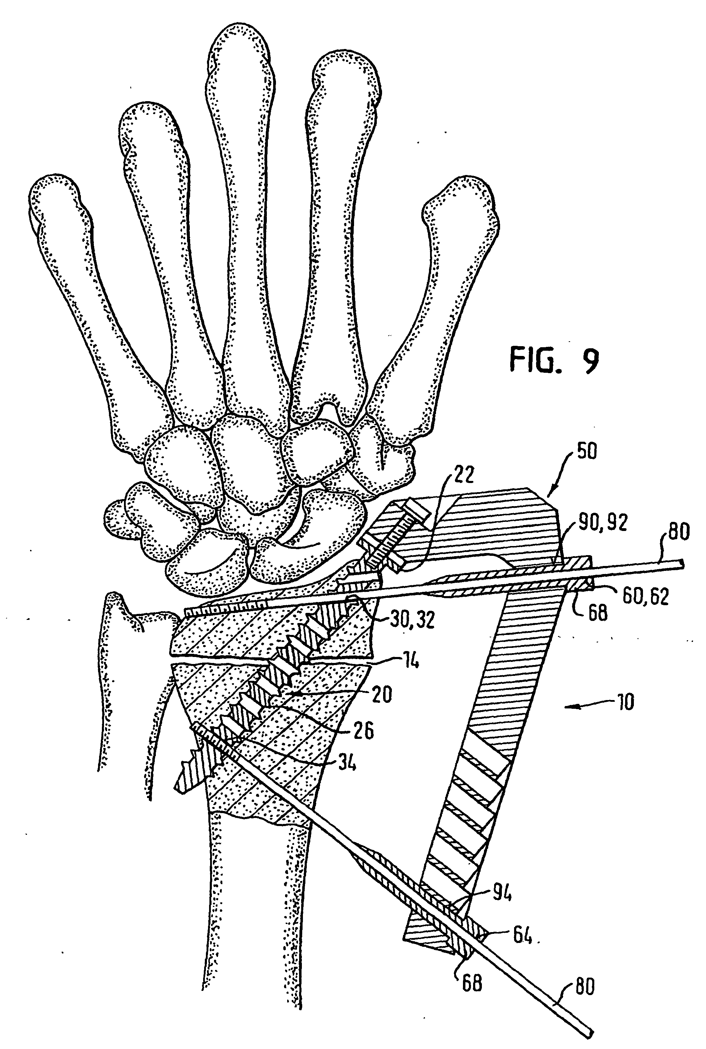

Anhand von Fig. 9 wird nun beispielhaft der Einsatz des erfindungsgemäßen Systems 10 bei der operativen Behandlung eines Bruches eines Unterarmknochens in der Nähe des Handgelenks beschrieben.

Die Fraktur wird zuerst unter Röntgendurchleuchtung geschlossen zurechtgelegt (ohne Operation), worauf ein kleiner Hautschnitt (ca. 1 cm) an der Spitze des Radiusknochens, gegen die Mittelhand herunter, auf der Seite des Daumens vorgenommen wird. Nach Vorbohrung unter einem vom Chirurgen zu wählenden Winkel zur Längsachse des Radiusknochens wird die Knochenschraube 20 über die Fraktur 14 eingeschraubt (ausgehend von der Radiusknochenspitze unterhalb der Fraktur am Handgelenk - die Fraktur durchkreuzend - und durch den Radiusknochen oberhalb der Fraktur näher am Ellbogen). Hierdurch wird eine Fixierung der Fraktur erhalten. Wenn die Frakturflächen gut erhalten sind (keine Quetschfraktur), kann im Bedarfsfalle eine Überbohrung mit etwas größerem Durchmesser des Schraubenlochs in dem nahe am Handgelenk gelegenen Frakturfragment ausgeführt werden, wodurch bewirkt wird, dass das Gewinde der Knochenschraube 20 in diesem Teil nicht eingreift, sondern nur auf der anderen Seite der Frakturlinie 14. Die eingeschraubte Knochenschraube 20 erzeugt dann eine Kompressionswirkung in der Frakturlinie 14, was die Stabilität der Fixierung verstärkt.

Nachdem die Schraube 20 angebracht worden ist, wird der Zielbügel 50 am Schraubenkopf 22 festgeschraubt. In die Aufnahmebohrungen 90, 92, 94 des Zielbügels 50 werden die Zielhülsen 68 eingebracht. Über Stechlöcher in der Haut können die Zielhülsen 68 durch die Zielbohrungen 60, 62, 64 im Zielbügel 50, durch die Haut hindurch und gegen den Knochen eingeführt werden. Über die Zielhülsen 68 können durchgehende Gewindestifte durch den nahe dem Zielbügel 50 gelegenen Knochen, durch die Fixierbohrungen 30, 32, 34 in der Knochenschraube 20 und durch den von dem Zielbügel 50 entfernten Knochen eingebohrt werden. Sobald die erforderliche Anzahl Gewindestifte 80 durch den Knochen und die Knochenschraube 20 eingeführt worden ist - in diesem Fall zwei Gewindestifte 80, jeweils einer auf jeder Seite der Fraktur - werden die Gewindestifte 80 neben dem Knochen abgeschnitten, der Zielbügel 50 wird entfernt und die kleinen Wunden werden zusammengenäht oder verbunden.

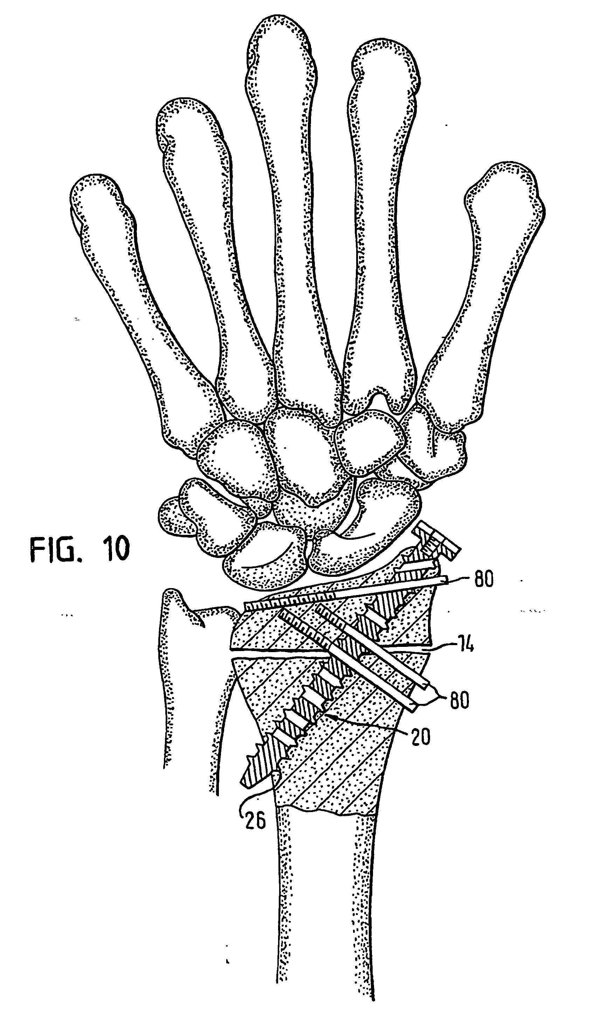

Fig. 10 zeigt einen Bruch eines Unterarmknochens in der Nähe des Handgelenks, der ähnlich wie bei Fig. 9. repositioniert (richtig gestellt) und mittels einer erfindungsgemäßen Knochenschraube 20 fixiert worden ist. Die Knochenschraube 20 ist mittels zweier Gewindestifte 80 auf der einen Seite und eines Gewindestifts 80 auf der anderen Seite der Frakturlinie 14 fertig fixiert worden. Die Stifte 80 sind gleich außerhalb des Knochens abgeschnitten.

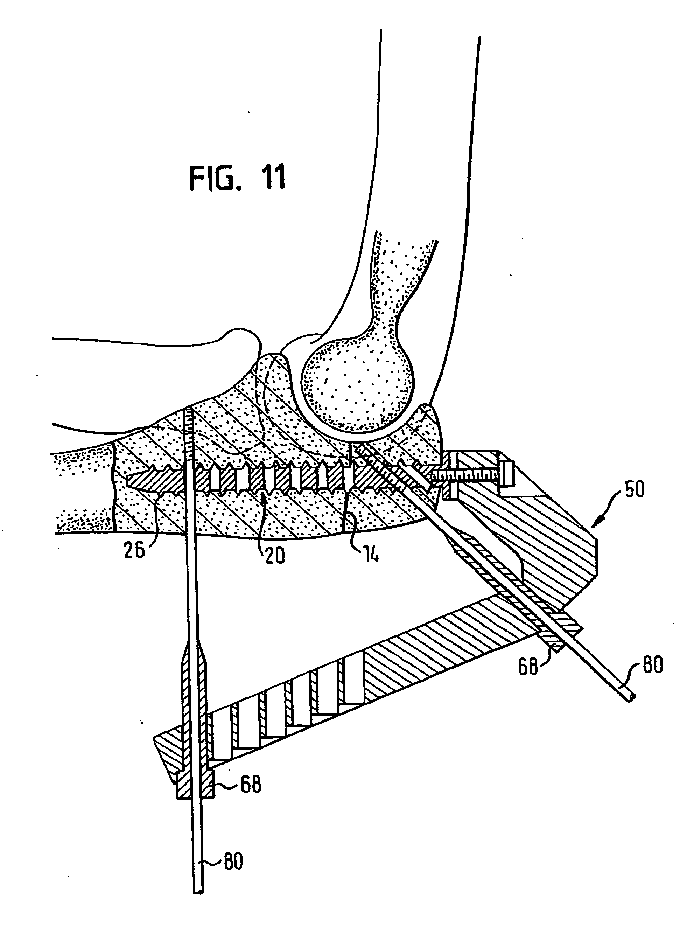

In Fig. 11 ist eine Fraktur im Olecranon (prox. Ulna) dargestellt, die repositioniert und mittels einer Knochenschraube 20 fixiert worden ist. Am Zielbügel 50 sind die Zielhülsen 68 angebracht und ein Gewindestift 80 ist beiderseits der Frakturlinie 14 eingebohrt.

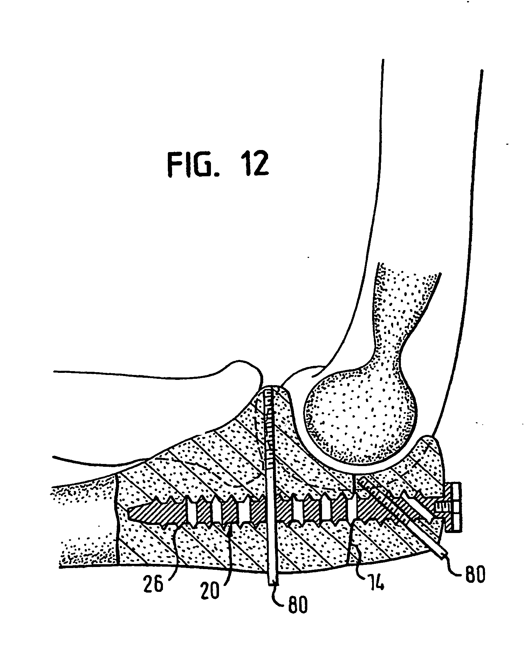

Fig. 12 veranschaulicht eine Fraktur in der Ellenbogenspitze ähnlich wie in Fig. 11, die mittels einer Knochenschraube 20 und zweier jeweils beiderseits der Frakturlinie 14 eingebrachter, abgeschnittener Gewindestifte 80 fertig fixiert worden ist.

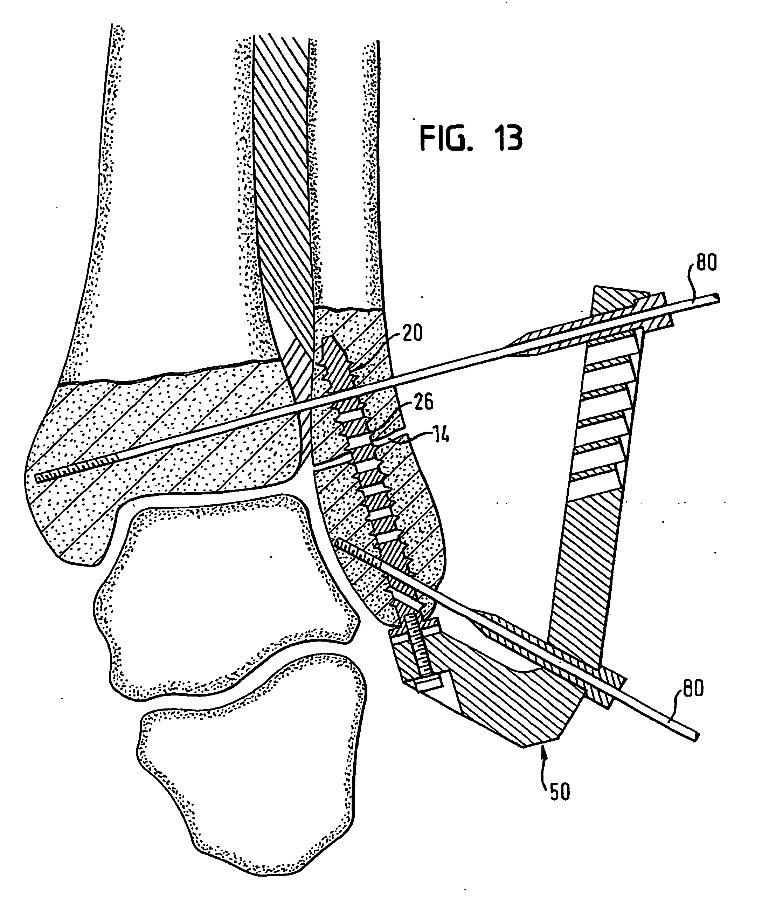

Eine Fraktur im äußeren Malleolus lateralis (distale Fibula), die repositioniert und mittels einer erfindungsgemäßen Knochenschraube 20, eines Zielbügels 50 und eines Gewindestifts 80 beiderseits der Frakturlinie 14 fertig fixiert worden ist, ist in Fig. 13 gezeigt.

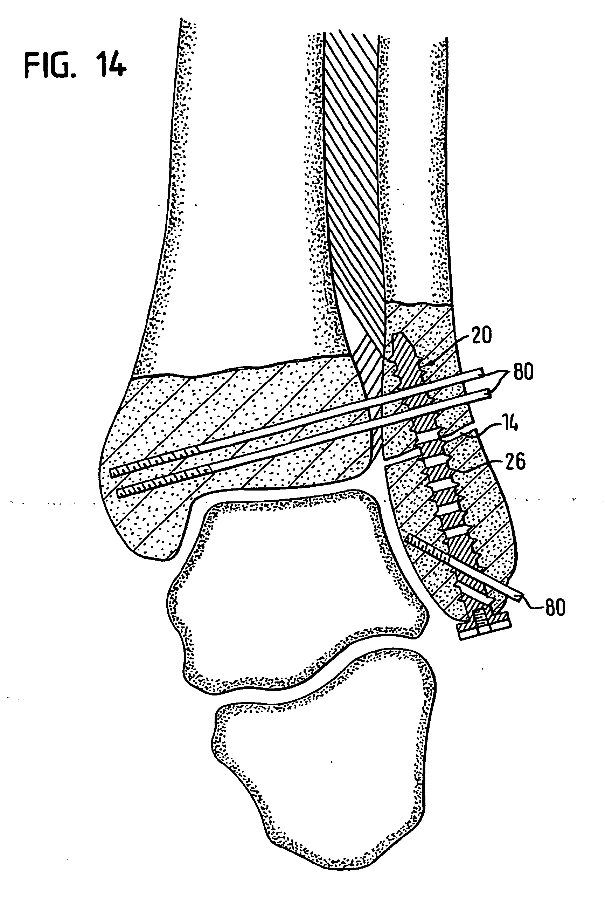

In Fig. 14 ist eine Fraktur im Malleolus lateralis ähnlich wie in Fig. 13 gezeigt, die mittels zweier abgeschnittener Gewindestifte 80 auf der einen Seite und eines Gewindestifts 80 auf der anderen Seite der Frakturlinie 14 fertig fixiert worden ist.

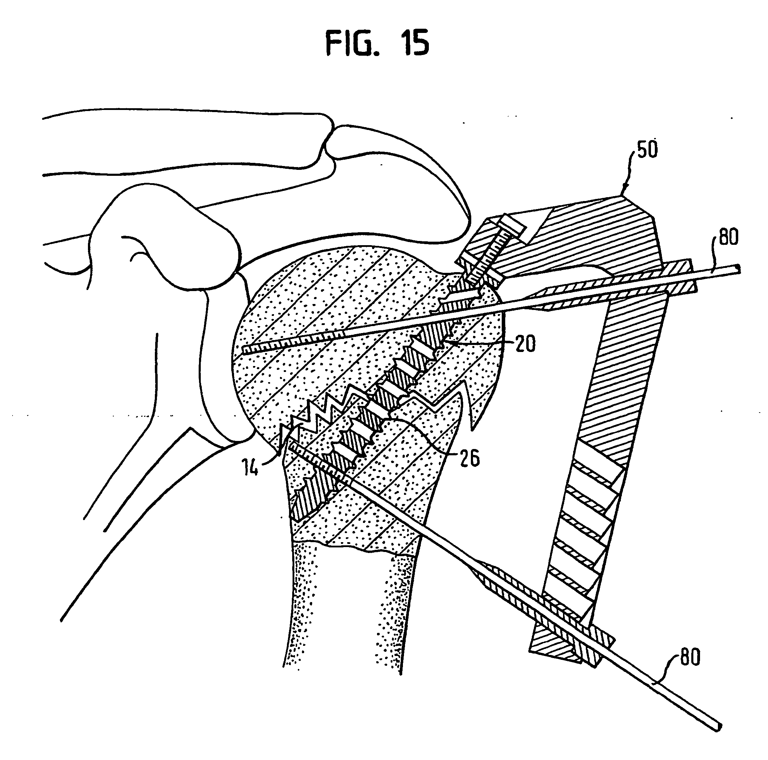

Die Darstellung in Fig. 15 veranschaulicht eine Fraktur im Schultergelenkkopf mit minimaler Fehlausrichtung, welche mittels einer von oben eingeführten erfindungsgemäßen Knochenschraube 20, eines Zielbügels 50 und eines Gewindestifts 80 beiderseits der Frakturlinie 14 fertig fixiert worden ist.

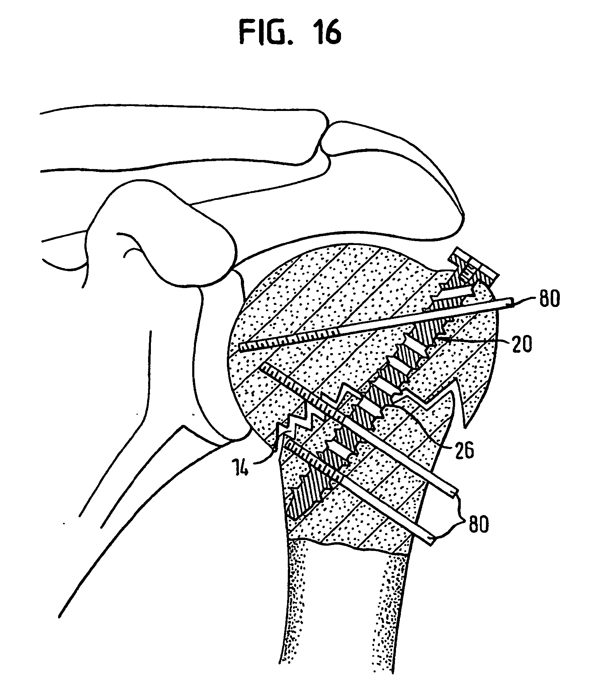

Fig. 16 zeigt eine Fraktur im Schultergelenkkopf ähnlich wie in Fig. 15, die mittels zweier abgeschnittener Gewindestifte 80 auf der einen Seite und eines Gewindestifts 80 auf der anderen Seite der Frakturlinie 14 fertig fixiert worden ist.

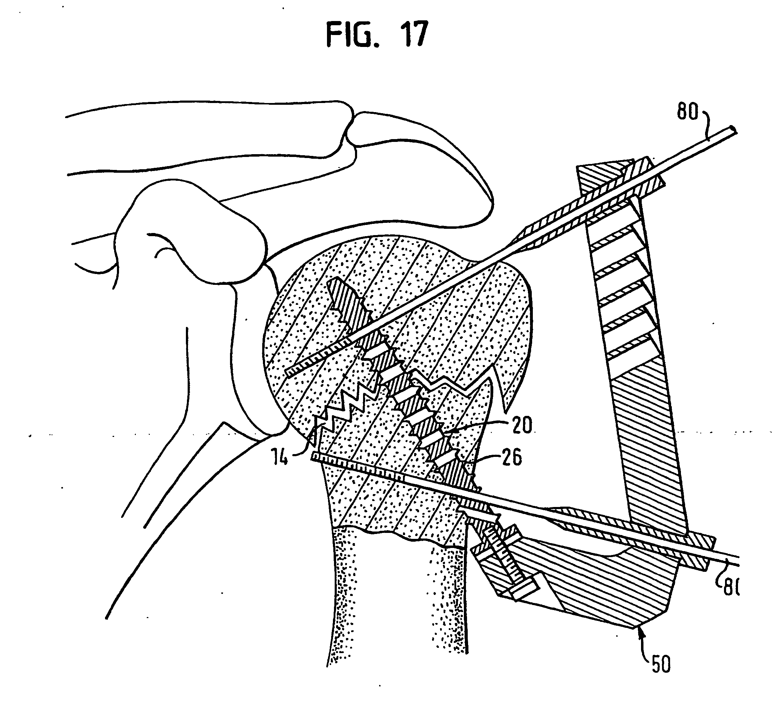

In Fig. 17 ist eine Fraktur im Schultergelenkkopf verdeutlicht, die mittels einer von unten eingeführten erfindungsgemäßen Knochenschraube 20, eines Zielbügels 50 und eines Gewindestifts 80 beiderseits der Frakturlinie 14 fertig fixiert worden ist.

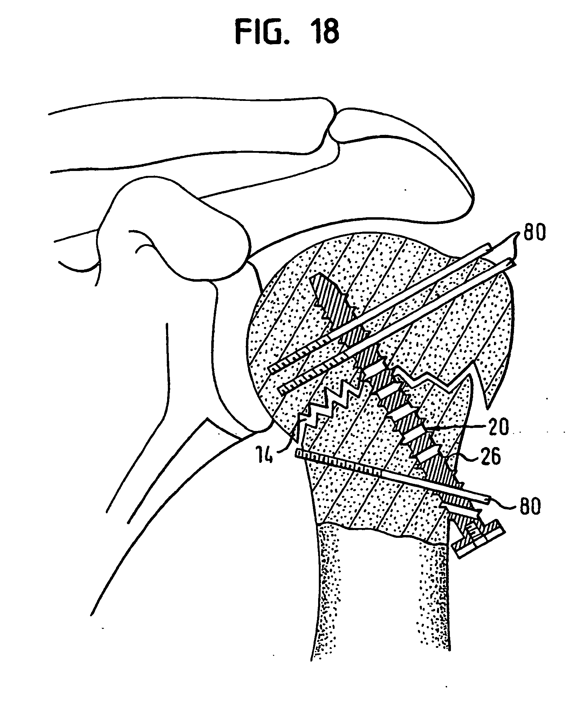

Fig. 18, die ähnlich wie Fig. 17 eine Fraktur im Schultergelenkkopf zeigt, veranschaulicht, wie die Knochenschraube 20 von unten eingeführt und über zwei abgeschnittene Gewindestifte 80 auf der einen Seite und einen Gewindestift 80 auf der anderen Seite der Frakturlinie 14 fertig fixiert worden ist.

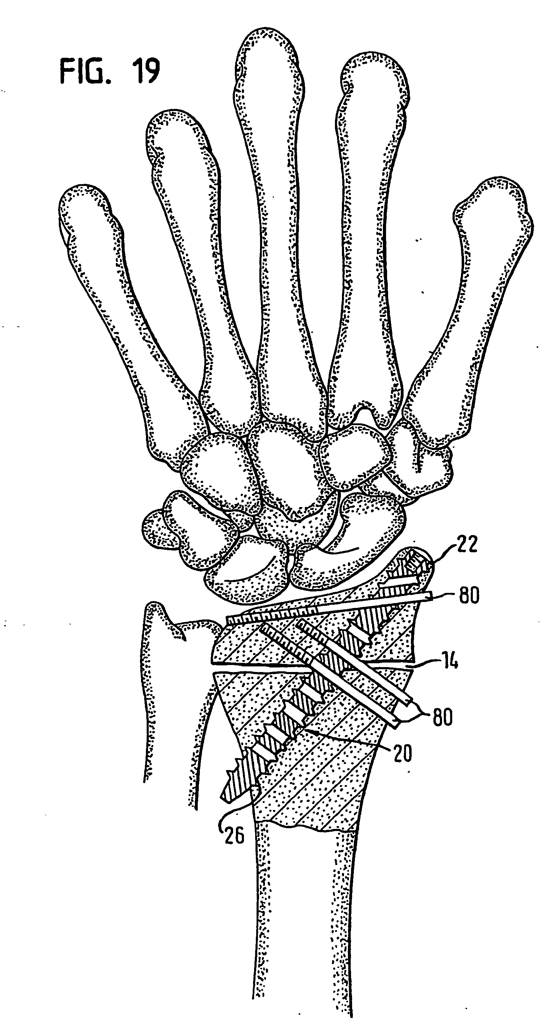

Fig. 19 zeigt einen Bruch eines Unterarmknochens in der Nähe des Handgelenks, der mittels einer modifizierten erfindungsgemäßen Knochenschraube 20 ohne radial hervorstehenden Schraubenkopf 22 und mittels zweier abgeschnittener Gewindestifte 80 die Fraktur überschreitend und eines Gewindestifts 80 auf einer Seite der Frakturlinie 14 fertig fixiert worden ist.

### Bezugszeichenliste

- 10: Knochenfixierungssystem
- 14: Frakturlinie
- 18: Unterseite Schraubenkopf
- 20: Knochenschraube
- 22: Schraubenkopf
- 24: Schaftabschnitt
- 26: Außengewindeabschnitt
- 28: Durchgangsbohrung
- 30: Fixierbohrung, Schrägbohrung
- 32: Fixierbohrung, Schrägbohrung
- 34: Fixierbohrung, Querbohrung
- 38: Schneidkante Gewinde
- 40: Kopplungseinrichtung
- 42: Gewindebohrung
- 44: Positioniereinrichtung, Positionierausnehmung
- 46: Innensechskant
- 48: Aussparung
- 50: Zielhilfe, Zielbügel
- 52: Zielabschnitt
- 54: Kopplungsabschnitt
- 56: Positioniereinrichtung, Positioniererhebung
- 58: Durchgangsloch
- 60: Zielbohrung
- 62: Zielbohrung
- 64: Zielbohrung
- 66: Zielhülsenabsatz
- 68: Zielhülse
- 70: Schraube
- 80: Fixierelement
- 90: Aufnahmebohrung
- 92: Aufnahmebohrung
- 94: Aufnahmebohrung
- 96: Flachsenkung
- 98: Flachsenkung
- A: Schraubenachse

## Patentansprüche

1. Knochenfixierungssystem zur Festlegung der Relativlage von Knochenfragmenten einer Fraktur, umfassend
- eine Knochenschraube (20) mit einem Schraubenkopf (22) und einem daran anschließenden Schaftabschnitt (24), der einen Außengewindeabschnitt (26) aufweist, über den die Knochenschraube (20) in den Knochen frakturüberschreitend einschraubbar ist, wobei am Schraubenkopf (22) eine Kopplungseinrichtung (40) vorgesehen ist und im Schaftabschnitt (24) mindestens zwei durch den Schaftabschnitt (24) verlaufende Fixierbohrungen (30, 32, 34) ausgebildet sind, und
- mindestens zwei stiftförmige Fixierelemente (80), die zur Fixierung der Knochenschraube (20) im Knochen durch die Fixierbohrung (30, 32) durchführbar sind
**dadurch gekennzeichnet, dass**
- von den zwei Fixierbohrungen (30, 32, 34) wenigstens eine als Schrägbohrung ausgebildet ist, die unter einem spitzen Winkel zur Schraubenachse (A) verläuft, und dass die beiden Fixierbohrungen (30, 32, 34) gegeneinander um die Schraubenachse (A) verdreht sind,
und durch
- eine extrakorporale Zielhilfe (50) mit einem Kopplungsabschnitt (54) und einem Zielabschnitt (52), wobei die Knochenschraube (20) und die Zielhilfe (50) über die Kopplungseinrichtung (40) des Schraubenkopfes (22) und den Kopplungsabschnitt (54) des Zielabschnittes (52) in genau einer vorgegebenen Relativlage miteinander koppelbar sind, und wobei im Zielabschnitt (52) mindestens zwei den Fixierbohrungen der Knochenschraube (20) zugeordnete und im gekoppelten Zustand mit diesen ausgerichtete Zielbohrungen (60, 62, 64) ausgebildet sind.

2. Knochenfixierungssystem nach Anspruch 1,
**dadurch gekennzeichnet, dass**
eine Fixierbohrung (30) als Schrägbohrung ausgebildet ist, die unter einem spitzen Winkel zur Schraubenachse (A) verläuft, und mindestens eine weitere Fixierbohrung (34) als Querbohrung ausgebildet ist und rechtwinklig zur Schraubenachse (A) verläuft, wobei der Schrägbohrung (30) und der Querbohrung (34) entsprechend ausgerichtete Zielbohrungen (60, 64) zugeordnet sind.

3. Knochenfixierungssystem nach Anspruch 1 oder 2,
**dadurch gekennzeichnet, dass**
mindestens zwei Fixierbohrungen (30, 32) als Schrägbohrungen ausgebildet sind, die unter einem spitzen Winkel zur Schraubenachse (A) verlaufen und gegeneinander um die Schraubenachse (A) verdreht sind, wobei den Schrägbohrungen (30, 32) entsprechend ausgerichtete Zielbohrungen (60, 62) zugeordnet sind, wobei insbesondere die beiden Schrägbohrungen (30, 32) zwischen dem Schraubenkopf (22) und wenigstens einer, bevorzugt mehreren und insbesondere parallel verlaufenden Querbohrungen (34) vorgesehen sind, wobei diesen Bohrungen (30, 32, 34) entsprechend ausgebildete Zielbohrungen (60, 62, 64) zugeordnet sind und/oder die beiden Schrägbohrungen (30, 32) nahe dem Schraubenkopf (22) und die Querbohrungen (34) im Endbereich des Schaftabschnittes (24) vorgesehen sind.

4. Knochenfixierungssystem nach einem der vorhergehenden Ansprüche,
**dadurch gekennzeichnet, dass**
die Kopplungseinrichtung (40) des Schraubenkopfes (22) eine in axialer Richtung der Knochenschraube (20) verlaufende Gewindebohrung (42) und eine Positioniereinrichtung (44) in Form einer an der Stirnseite des Schraubenkopfes (22) vorgesehenen Positionierausnehmung umfasst, und dass der Kopplungsabschnitt (54) der Zielhilfe (50) ein Durchgangsloch (58) und eine Positioniereinrichtung (56) in Form einer zur Positionierausnehmung komplementären Positioniererhebung umfasst, wobei insbesondere die Positionierausnehmung (44) eine sich radial verjüngende Nut oder zumindest eine durch die Schraubenachse (A) verlaufende kerben- oder schlitzartige Vertiefung umfasst, wobei vorzugsweise zusätzlich zu der kerben- oder schlitzartigen Vertiefung eine randseitig am Schraubenkopf (22) angeordnete Aussparung (48) ausgebildet ist und/oder bei nicht gekoppelter Zielhilfe (50) an die Positioniereinrichtung (44) der Knochenschraube (20) ein Eindrehwerkzeug ansetzbar ist, und/oder an der Stirnseite des Schraubenkopfes (22) ein Innensechskant (46) als Aufnahme für ein Eindrehwerkzeug vorgesehen ist.

5. Knochenfixierungssystem nach einem der vorhergehenden Ansprüche,
**dadurch gekennzeichnet, dass**
die Zielhilfe (50) als Zielbügel mit einstückig ausgebildetem Kopplungsabschnitt (54) und Zielabschnitt (52) gestaltet ist.

6. Knochenfixierungssystem nach einem der vorhergehenden Ansprüche,
**dadurch gekennzeichnet, dass**
der Zielabschnitt (52) der Zielhilfe (50) und die Knochenschraube (20) im gekoppelten Zustand in einer Ebene liegen und jeweils auseinanderlaufende Schenkel eines U oder V bilden, wobei die Schenkel bevorzugt zumindest näherungsweise gleich lang sind.

7. Knochenfixierungssystem nach einem der vorhergehenden Ansprüche,
**dadurch gekennzeichnet, dass**
die Knochenschraube (20) eine in axialer Richtung verlaufende Durchgangsbohrung (28) aufweist, und/oder eine im eingeschraubten Zustand dem Knochen zugewandte Unterseite (18) des Schraubenkopfes (22) als an die Form des jeweiligen Knochens angepasste Anlagefläche ausgebildet ist.

8. Knochenfixierungssystem nach einem der vorhergehenden Ansprüche,
**dadurch gekennzeichnet, dass**
die Knochenschraube (20) einen Gewindedurchmesser zwischen 4 mm und 12 mm, vorzugsweise zwischen 5 mm und 8 mm, aufweist, und/oder die Knochenschraube (20) eine Länge zwischen 3 cm und 15 cm, vorzugsweise zwischen 4 cm und 10 cm, aufweist, und/oder die Knochenschraube (20) ein mehrgängiges, vorzugsweise zweigängiges, Außengewinde aufweist, und/oder die Steigung des Gewindes der Knochenschraube (20) zwischen 1 mm und 3 mm, vorzugsweise etwa 2 mm, pro Gang beträgt, und/oder die Knochenschraube (20) ein selbstschneidendes Gewinde aufweist, und/oder die Fixierbohrungen (30, 32, 34) einen Durchmesser zwischen 1 mm und 3 mm, vorzugsweise zwischen 1,6 mm und 2 mm, aufweisen.

9. Knochenfixierungssystem nach einem der vorhergehenden Ansprüche,
**dadurch gekennzeichnet , dass**
die Knochenschraube (20) aus einem biokompatiblen Material, vorzugsweise Titan, besteht, und/oder die Knochenschraube (20) aus einem vom Körper wieder aufnehmbaren Material, beispielsweise Polyglykolid oder Poly-L-Laktid, besteht.

10. Knochenfixierungssystem nach einem der vorhergehenden Ansprüche,
**dadurch gekennzeichnet, dass**
in dem Zielabschnitt (52) zumindest eine Aufnahmebohrung (90, 92, 94) vorgesehen ist, in die eine Zielhülse (68) mit einer darin ausgebildeten Zielbohrung (60, 62, 64) einbringbar, vorzugsweise steckbar, ist, die mit der Fixierbohrung (30, 32, 34) ausgerichtet und in Richtung der Fixierbohrung (30, 32, 34) verschiebbar ist.

11. Knochenfixierungssystem nach einem der vorhergehenden Ansprüche,
**dadurch gekennzeihnet,** dass
die Zielhilfe (50) zumindest im Bereich des Kopplungsabschnittes (54) aus für Röntgenstrahlen durchlässigem Material, beispielsweise kohlefaserverstärktem Kunststoff, besteht, und/oder das Fixierelement (80) eine Schraube oder ein Nagel ist.

12. Knochenfixierungssystem nach einem der vorhergehenden Ansprüche,
**dadurch gekennzeichnet, dass**
die Zielhilfe (50) universell ist und mit Knochenschrauben (20) mit unterschiedlichen Längen und/oder unterschiedlichen Anzahlen und Lagen von Fixierbohrungen (30, 32, 34) verwendbar ist.

13. Knochenschraube für ein Knochenfixierungssystem nach einem der vorhergehenden Ansprüche, mit einem Schraubenkopf (22) und einem daran anschließenden Schaftabschnitt (24), der einen Außengewindeabschnitt (26) aufweist, über den die Knochenschraube (20) in den Knochen frakturüberschreitend einschraubbar ist,
**dadurch gekennzeichnet,**
**dass** am Schraubenkopf (22) eine Kopplungseinrichtung (40) zur Kopplung mit der Zielhilfe (50) vorgesehen ist und im Schaftabschnitt (24) mindestens zwei durch den Schaftabschnitt (24) verlaufende Fixierbohrungen (30, 32, 34) ausgebildet sind, wobei von den zwei Fixierbohrungen (30, 32, 34) mindestens eine als Schrägbohrung ausgebildet ist, deren Längsachse unter einem spitzen Winkel zur Schraubenachse (A) verläuft, und die beiden Fixierbohrungen (30, 32, 34) gegeneinander um die Schraubenachse (A) verdreht sind.

14. Knochenschraube nach Anspruch 13 mit den eine Knochenschraube betreffenden Merkmalen zumindest eines der Ansprüche 2 bis 12.

15. Zielhilfe für ein Knochenfixierungssystem nach einem der vorhergehenden Ansprüche, mit einem Kopplungsabschnitt (54) zur Kopplung mit einer Knochenschraube (20) nach Anspruch 13 oder 14 und einem Zielabschnitt (52), wobei die Zielhilfe (50) über den Kopplungsabschnitt (54) in genau einer vorgegebenen Relativlage mit der Knochenschraube (20) koppelbar ist, und wobei im Zielabschnitt (52) mindestens zwei Zielbohrungen (60, 62) ausgebildet sind, die den Fixierbohrungen der Knochenschraube (20) zugeordnet sind und im mit der Knochenschraube (20) gekoppelten Zustand mit diesen ausgerichtet sind,
**dadurch gekennzeichnet,**
**dass** von den zwei Zielbohrungen (60, 62, 64) wenigstens eine als eine solche Bohrung ausgebildet ist, deren Längsachse im mit der Knochenschraube (20) gekoppelten Zustand unter einem spitzen Winkel zu einer der Längsachse (A) der Knochenschraube (20) entsprechenden Achse (A) verläuft, und die beiden Zielbohrungen (60, 62, 64) gegeneinander um die der Längsachse (A) der Knochenschraube (20) entsprechende Achse (A) verdreht sind.

16. Zielhilfe nach Anspruch 15 mit den eine Zielhilfe betreffenden Merkmalen zumindest eines der Ansprüche 2 bis 12.

## Claims

1. A bone fixation system for the fixing of the relative position of bone fragments of a fracture, comprising
- a bone screw (20) having a screw head (22) and a shank section (24) adjoining it which has an external thread section (26) via which the bone screw (20) can be screwed into the bone in a fracture-crossing manner, wherein a coupling device (40) is provided at the screw head (22) and at least two fixation bores (30, 32, 34) extending through the shank section (24) are formed in the shank section (24); and
- at least two pin-like fixation elements (80) which can be led through the fixation bore (30, 3,) for the fixation of the bone screw (20) in the bone,
**characterised in that**
- at least one of the two fixation bores (30, 32, 34) is made as an inclined bore which extends at an acute angle to the axis of the screw (A); and **in that** the two fixation bores (30, 32, 34) are rotated relative to one another about the axis of the screw (A);
and by
- an extra-corporeal targeting aid (50) having a coupling section (54) and a target section (52), wherein the bone screw (20) and the targeting aid (50) can be coupled with one another in exactly one pre-determined relative position via the coupling device (40) of the screw head (22) and the coupling section (54) of the target section (52), and wherein at least two target bores (60, 62, 64) are formed in the target section (52), are associated with the fixation bores of the bone screw (20) and are aligned therewith in the coupled state.

2. A bone fixation system in accordance with claim 1, **characterised in that** a fixation bore (30) is made as an inclined bore which extends at an acute angle to the axis of the screw (A); and **in that** at least one further fixation bore (34) is made as a transverse bore and extends at a right angle to the axis of the screw (A), wherein the inclined bore (30) and the transverse bore (34) are associated with correspondingly aligned target bores (60 64).

3. A bone fixation system in accordance with claim 1 or claim 2, **characterised in that** at least two fixation bores (30, 32) are made as inclined bores which extend at an acute angle to the axis of the screw (A) and are rotated relative to one another about the axis of the screw (A), wherein the inclined bores (30, 32) are associated with correspondingly aligned target bores (60, 62), wherein in particular the two inclined bores (30, 32) are provided between the screw head (22) and at least one transverse bore (34), preferably a plurality of transverse bores (34), which in particular extend in parallel, wherein these bores (30, 32, 34) are associated with correspondingly made target bores (60, 62, 64) and/or the two inclined bores (30, 32) are provided close to the screw head (22) and the transverse bores (34) are provided in the end region of the shank section (24).

4. A bone fixation system in accordance with any one of the preceding claims, **characterised in that** the coupling device (40) of the screw head (22) includes a tapped bore (42) extending in the axial direction of the bone screw (20) and a positioning device (44) in the form of a positioning cut-out provided at the end face of the screw head (22); and **in that** the coupling section (54) of the targeting aid (50) includes a passage bore (58) and a positioning device (56) in the form of a positioning elevation complementary to a positioning cut-out, wherein the positioning cut-out (44) in particular includes a radially tapering groove, or at least a notch-like or slit-like recess extending through the axis of the screw (A), wherein a cut-out (48) arranged at the rim side at the screw head (22) is preferably formed in addition to the notch-like or slit-like recess and/or wherein a screw-in tool can be placed onto the positioning device (44) of the bone screw (20) with the targeting aid (50) not coupled and/or wherein a hexagonal socket (46) is provided at the end face of the screw head (22) as a receiver for a screw-in tool.

5. A bone fixation system in accordance with any one of the preceding claims, **characterised in that** the targeting aid (50) is designed as a targeting device or yoke with a coupling section (54) and a target section (52) formed in one piece.

6. A bone fixation system in accordance with any one of the preceding claims, **characterised in that** the target section (52) of the targeting aid (50) and the bone screw (20) form limbs of a U or of V in the coupled state which lie in one plane and diverge from one another, wherein the limbs are preferably at least approximately of the same length.

7. A bone fixation system in accordance with any one of the preceding claims, **characterised in that** the bone screw (20) has a passage bore (28) extending in an axial direction; and/or a lower side (18) of the screw head (22) facing the bone in the screwed-in state is formed as an abutment area matched to the shape of the respective bone.

8. A bone fixation system in accordance with any one of the preceding claims, **characterised in that** the bone screw (20) has a thread diameter between 4 mm and 12 mm, preferably between 5 mm and 8 mm; and/or the bone screw (20) has a length between 3 cm and 15 cm, preferably between 4 cm and 10 cm; and/or the bone screw (20) has a multiple thread, preferably a two-start, external thread, and/or the pitch of the thread of the bone screw (20) amounts to between 1 mm and 3 mm, preferably approximately to 2 mm, per thread; and/or the bone screw (20) has a self-tapping thread; and/or the fixation bores (30, 32, 34) have a diameter between 1 mm and 3 mm, preferably between 1.6 mm and 2 mm.

9. A bone fixation system in accordance with any one of the preceding claims, **characterised in that** the bone screw (20) consists of a biocompatible material, preferably titanium; and/or the bone screw (20) consists of a material which can be reabsorbed by the body again, for example polyglycolide or poly(L-lactide).

10. A bone fixation system in accordance with any one of the preceding claims, **characterised in that** at least one receiver bore (90, 92, 94) is provided in the target section (52) and a target sleeve (68) with a target bore (60, 62, 64) formed therein can be introduced, preferably plugged, therein, and is aligned with the fixation bore (30, 32, 34) and is displaceable in the direction of the fixation bore (30, 32, 34).

11. A bone fixation system in accordance with any one of the preceding claims, **characterised in that** the targeting aid (50) consists, at least in the region of the coupling section (54), of material permeable to X-rays, for example carbon fibre reinforced plastic; and/or the fixation element (80) is a screw or a nail.

12. A bone fixation system in accordance with any one of the preceding claims, **characterised in that** the targeting aid (50) is universal and can be used with bone screws (20) of different lengths and/or with different numbers and positions of fixation bores (30, 32, 34).

13. A bone screw for a bone fixation system in accordance with any one of the preceding claims, having a screw head (22) and a shank section (24) which adjoins it and has an external thread section (26) via which the bone screw (20) can be screwed into the bone in a fracture-crossing manner,
**characterised in that**
a coupling device (40) is provided at the screw head (22) for coupling with the targeting aid (50) and at least two fixation bores (30, 32, 34) extending through the shank section (24) are formed in the shank section (24), wherein at least one of the two fixation bores (30, 32, 34) is made as an inclined bore whose longitudinal axis extends at an acute angle to the axis of the screw (A) and wherein the two fixation bores (30, 32, 34) are rotated relative to one another about the axis of the screw (A).

14. A bone screw in accordance with claim 13 comprising the features relating to a bone screw of at least one of the claims 2 to 12.

15. A targeting aid for a bone fixation system in accordance with any one of the preceding claims comprising a coupling section (54) for coupling with a bone screw (20) in accordance with claim 13 or claim 14 and a target section (52), wherein the target aid (50) can be coupled with the bone screw (20) in exactly one pre-determined relative position via the coupling section (54), and wherein at least two target bores (60, 62, 64) are formed in the target section (52) and are associated with the fixation bores of the bone screw (20) and are aligned therewith in the state coupled with the bone screw (20),
**characterised in that**
at least one of the two target bores (60, 62, 64) is made as a bore of the kind whose longitudinal axis extends, in the state coupled with the bone screw (20) at an acute angle to an axis (A) corresponding to the longitudinal axis (A) of the bone screw (20) and wherein the two target bores (60, 62, 64) are rotated relative to one another about the axis (A) corresponding to the longitudinal axis (A) of the bone screw (20)

16. A targeting aid in accordance with claim 15, with the features relating to a targeting aid of at least one of the claims 2 to 12.

## Revendications

1. Système d'ostéosynthèse destiné à la fixation de la position relative de fragments d'os d'une fracture, comportant :
- une vis d'os (20) avec une tête de vis (22) et un tronçon de tige (24) s'y raccordant, qui est muni d'un tronçon à filetage extérieur (26) par l'intermédiaire duquel la vis d'os (20) peut être vissée dans l'os à travers la fracture, un dispositif de couplage (40) étant prévu sur la tête de vis (22), et au moins deux perçages de fixation (30, 32, 34) s'étendant à travers le tronçon de tige (24) étant pratiqués dans le tronçon de tige (24), et
- au moins deux éléments de fixation (80) en forme de broche qui peuvent être engagés à travers le perçage de fixation (30, 32) pour la fixation de la vis d'os (20) dans l'os,
**caractérisé en ce que**,
- des deux perçages de fixation (30, 32, 34), au moins un est agencé sous la forme d'un perçage oblique qui s'étend suivant un angle aigu par rapport à l'axe de vis (A), et **en ce que** les deux perçages de fixation (30, 32, 34) sont décalés l'un par rapport à l'autre autour de l'axe de vis (A),
et **en ce que**
- il est prévu une aide de visée (50) extracorporelle avec un tronçon de couplage (54) et un tronçon de visée (52), la vis d'os (20) et l'aide de visée (50) pouvant être couplées entre elles dans exactement une position relative prédéterminée par l'intermédiaire du dispositif de couplage (40) de la tête de vis (22) et du tronçon de couplage (54) du tronçon de visée (52), et au moins deux perçages de visée (60, 62, 64) associés aux perçages de fixation de la vis d'os (20), et alignés avec ceux-ci à l'état couplé, étant pratiqués dans le tronçon de visée (52).

2. Système d'ostéosynthèse selon la revendication 1,
**caractérisé en ce que**
un perçage de fixation (30) est agencé sous la forme d'un perçage oblique qui s'étend suivant un angle aigu par rapport à l'axe de vis (A), et **en ce qu'**au moins un autre perçage de fixation (34) est agencé sous la forme d'un perçage transversal et s'étend à angle droit par rapport à l'axe de vis (A), des perçages de visée (60, 64) alignés de façon correspondante étant associés au perçage oblique (30) et au perçage transversal (34).

3. Système d'ostéosynthèse selon la revendication 1 ou 2,
**caractérisé en ce que**
au moins deux perçages de fixation (30, 32) sont agencés sous la forme de perçages obliques qui s'étendent suivant un angle aigu par rapport à l'axe de vis (A), et qui sont décalés l'un par rapport à l'autre autour de l'axe de vis (A), des perçages de visée (60, 62) alignés de façon correspondante étant associés aux perçages obliques (30, 32), les deux perçages obliques (30, 32) étant notamment prévus entre la tête de vis (22) et au moins un, de préférence plusieurs, perçages transversaux (34), et s'étendant notamment parallèlement, des perçages de visée (60, 62, 64) agencés de façon correspondante étant associés à ces perçages (30, 32, 34), et/ou les deux perçages obliques (30, 32) étant prévus à proximité de la tête de vis (22) et les perçages transversaux (34) dans la zone d'extrémité du tronçon de tige (24).

4. Système d'ostéosynthèse selon l'une des revendications précédentes,
**caractérisé en ce que**
le dispositif de couplage (40) de la tête de vis (22) comporte un perçage taraudé (42) s'étendant dans la direction axiale de la vis d'os (20), et un dispositif de positionnement (44) sous la forme d'un évidement de positionnement prévu sur la face frontale de la tête de vis (22), et **en ce que** le tronçon de couplage (54) de l'aide de visée (50) comporte un orifice de passage (58) et un dispositif de positionnement (56) sous la forme d'une surélévation de positionnement complémentaire à l'évidement de positionnement, l'évidement de positionnement (44) comportant notamment une rainure se rétrécissant radialement ou au moins un renfoncement de type encoche ou fente passant par l'axe de vis (A), un évidement (48) agencé du côté du bord de la tête de vis (22) étant de préférence prévu en plus du renfoncement de type encoche ou fente, et/ou un outil de vissage pouvant être rapporté au dispositif de positionnement (44) de la vis d'os (20) à l'état non couplé de l'aide de visée (50), et/ou un six pans creux (46) étant prévu sur la face frontale de la tête de vis (22) en tant que réception pour un outil de vissage.

5. Système d'ostéosynthèse selon l'une des revendications précédentes,
**caractérisé en ce que**
l'aide de visée (50) est agencée sous la forme d'un étrier de visée avec un tronçon de couplage (54) et un tronçon de visée (52) réalisés d'un seul tenant.

6. Système d'ostéosynthèse selon l'une des revendications précédentes,
**caractérisé en ce que**,
à l'état couplé, le tronçon de visée (52) de l'aide de visée (50) et la vis d'os (20) sont situés dans un plan, et forment respectivement des branches d'un U ou d'un V s'écartant l'une de l'autre, les branches étant de préférence au moins approximativement de même longueur.

7. Système d'ostéosynthèse selon l'une des revendications précédentes,
**caractérisé en ce que**
la vis d'os (20) comporte un perçage de passage (28) s'étendant dans la direction axiale, et/ou **en ce qu'**une face inférieure (18) de la tête de vis (22) orientée à l'état vissé vers l'os est agencée en tant que surface d'appui adaptée à la forme de l'os respectif.

8. Système d'ostéosynthèse selon l'une des revendications précédentes,
**caractérisé en ce que**
la vis d'os (20) possède un diamètre de filetage compris entre 4 mm et 12 mm, de préférence entre 5 mm et 8 mm, et/ou **en ce que** la vis d'os (20) possède une longueur comprise entre 3 cm et 15 cm, de préférence entre 4 cm et 10 cm, et/ou **en ce que** la vis d'os (20) possède un filetage extérieur à filets multiples, de préférence à filet double, et/ou **en ce que** le pas du filetage de la vis d'os (20) est compris entre 1 mm et 3 mm par filet, et est de préférence de l'ordre de 2 mm, et/ou **en ce que** la vis d'os (20) comporte un filetage auto-taraudeur, et/ou **en ce que** les perçages de fixation (30, 32, 34) ont un diamètre compris entre 1 mm et 3 mm, de préférence entre 1,6 mm et 2 mm.

9. Système d'ostéosynthèse selon l'une des revendications précédentes,
**caractérisé en ce que**
la vis d'os (20) est constituée d'un matériau biocompatible, de préférence de titane, et/ou **en ce que** la vis d'os (20) est constituée d'un matériau pouvant également être accepté par le corps, par exemple de polyglycolide ou de poly-L-lactide.

10. Système d'ostéosynthèse selon l'une des revendications précédentes,
**caractérisé en ce que**
au moins un perçage de réception (90, 92, 94) est prévu dans le tronçon de visée (52), dans lequel peut être insérée, de préférence enfichée, une douille de visée (68) dans laquelle est pratiqué un perçage de visée (60, 62, 64), qui est aligné avec le perçage de fixation (30, 32, 34), et qui peut être déplacée en direction du perçage de fixation (30, 32, 34).

11. Système d'ostéosynthèse selon l'une des revendications précédentes,
**caractérisé en ce que**
l'aide de visée (50) est constituée d'un matériau perméable aux rayons X, au moins dans la zone du tronçon de couplage (54), par exemple de matière plastique renforcée par des fibres de carbone, et/ou **en ce que** l'élément de fixation (80) est une vis ou un clou.

12. Système d'ostéosynthèse selon l'une des revendications précédentes,
**caractérisé en ce que**
l'aide de visée (50) est universelle, et peut être utilisée avec des vis d'os (20) de différentes longueurs et/ou avec différents nombres et positions de perçages de fixation (30, 32, 34).

13. Vis d'os pour un système d'ostéosynthèse selon l'une des revendications précédentes, comportant une tête de vis (22) et un tronçon de tige (24) s'y raccordant, qui est muni d'un tronçon à filetage extérieur (26) par l'intermédiaire duquel la vis d'os (20) peut être vissée dans l'os à travers la fracture,
**caractérisée en ce que**
un dispositif de couplage (40) est prévu sur la tête de vis (22) pour le couplage avec l'aide de visée (50), et **en ce qu'**au moins deux perçages de fixation (30, 32, 34) s'étendant à travers le tronçon de tige (24) sont pratiqués dans le tronçon de tige (24), au moins un des deux perçages de fixation (30, 32, 34) étant agencé sous la forme d'un perçage oblique dont l'axe longitudinal s'étend suivant un angle aigu par rapport à l'axe de vis (A), et les deux perçages de fixation (30, 32, 34) étant décalés l'un par rapport à l'autre autour de l'axe de vis (A).

14. Vis d'os selon la revendication 13 présentant les particularités relatives à une vis d'os de l'une au moins des revendications 2 à 12.

15. Aide de visée pour un système d'ostéosynthèse selon l'une des revendications précédentes, comportant un tronçon de couplage (54) pour le couplage à une vis d'os (20) selon la revendication 13 ou 14, et un tronçon de visée (52), l'aide de visée (50) pouvant être couplée à la vis d'os (20) dans exactement une position relative prédéterminée par le tronçon de couplage (54), et au moins deux perçages de visée (60, 62) étant pratiqués dans le tronçon de visée (52), lesquels sont associés aux perçages de fixation de la vis d'os (20) et alignés avec ceux-ci à l'état couplé à la vis d'os (20), **caractérisée en ce que**,
des deux perçages de visée (60, 62, 64), au moins un est agencé sous la forme d'un perçage dont l'axe longitudinal s'étend à l'état couplé avec la vis d'os (20) suivant un angle aigu par rapport à un axe (A) correspondant à l'axe longitudinal (A) de la vis d'os (20), et les deux perçages de visée (60, 62, 64) étant décalés l'un par rapport à l'autre autour de l'axe (A) correspondant à l'axe longitudinal (A) de la vis d'os (20).

16. Aide de visée selon la revendication 15 présentant les particularités relatives à une aide de visée de l'une au moins des revendications 2 à 12.
